# DEMANDE DE BREVET EUROPEEN

(11) **EP 0 842 921 A1**
(43) Date de publication de la demande: **20.05.1998**
(21) Numéro de dépôt: 97402635.3
(22) Date de dépôt: 05.11.1997
(51) Int. Cl.: C07C 213/04, C07C 217/28

(54) **Procédé de préparation d'amines polyglycérylées, les amines ainsi obtenues et leurs dérivés**

(30) Priorité: 14.11.1996 FR 9613907
(71) Demandeur: CECA S.A., 92800 Puteaux (FR)
(72) Inventeur: Rivaux, Yvan, 35700 Rennes (FR); Noiret, Nicolas, 35250 Saint Sulpice La Foret (FR); Patin, Henri, 35200 Rennes (FR); Fouquay, Stéphane, 76130 Mont Saint Aignan (FR); Laffitte, Jean-Alex, 64000 Pau (FR)
(74) Mandataire: Haicour, Philippe

(57) **Abrégé**

L'invention concerne un procédé de préparation d'amines polyglycérylées de formule : 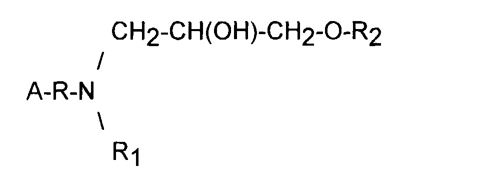 dans laquelle R représente une chaîne hydrocarbonée, linéaire ou ramifiée, saturée ou insaturée, en C₆-C₃₂, R₁ représente une chaîne hydrocarbonée, linéaire ou ramifiée, saturée ou insaturée, en C₁-C₂₂ ou -CH₂-CH(OH)-CH₂-O-R₂, A représente H ou 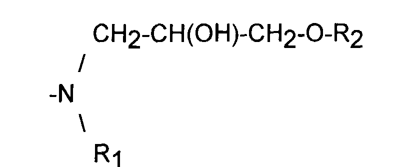 R₂ représente un motif ou un enchaînement de motifs de formule : 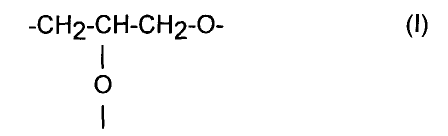 et/ou 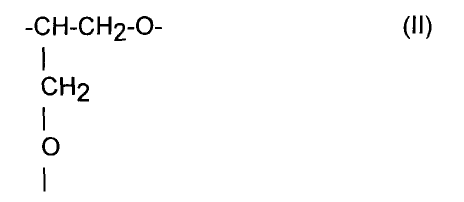 formule dans laquelle la valence disponible de l'atome d'oxygène est liée à un atome d'hydrogène,
ledit procédé étant caractérisé en ce qu'il consiste à :
***a***) faire réagir une amine de formule 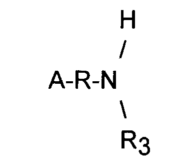 et un éther de formule : 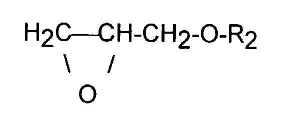 dans laquelle R₃ représente H ou une chaîne hydrocarbonée, linéaire ou ramifiée, saturée ou insaturée, en C₁-C₂₂, et A, R, R₂ ont la signification donnée ci-avant, la valence disponible de l'atome d'oxygène étant liée à un groupement protecteur, et
***b)*** déprotéger.

Elle a également pour objet les amines précitées et leurs dérivés se présentant notamment sous la forme d'oxyde d'amine, d'ammonium quaternaire, de sel d'ammonium et de bétaïne.

## Description

La présente invention concerne un procédé de préparation d'amines polyglycérylées, les amines ainsi obtenues et leurs dérivés notamment sous la forme d'oxyde d'amine, d'ammonium quaternaire, de sel d'ammonium et de bétaïne.

Les amines tertiaires polyglycérylées sont connues pour leurs propriétés tensioactives. Elles sont généralement préparées par réaction d'une amine aliphatique primaire ou secondaire et de glycidol (voir US 2 784 233 ; JP 58-63736 ; ULSPERGER E. et DEHNS R., Journal für praktische Chemie, vol. 4, n°27, pp. 195-212, 1965).

Dans les procédés précités, on obtient des amines contenant une ou deux chaînes glycérylées linéaires formées par l'enchaînement de motifs glycérol liés en positions 1 et 3, ledit enchaînement étant formé de manière statistique. Ces amines se présentent sous la forme d'un mélange complexe et leur isolement nécessite la mise en place d'opérations de fractionnement et de purification difficiles et coûteuses.

La présente invention propose un nouveau procédé de préparation d'amines polyglycérylées qui permet de contrôler le nombre de motifs glycérol des chaînes polyols et de former ainsi des composés monodispersés.

Plus précisément, ce procédé consiste à :
***a***) faire réagir une amine de formule 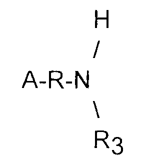 dans laquelle R représente une chaîne hydrocarbonée, linéaire ou ramifiée, saturée ou insaturée, en C₆-C₃₂, R₃ représente H ou une chaîne hydrocarbonée, linéaire ou ramifiée, saturée ou insaturée, en C₁-C₂₂ et A représente H ou 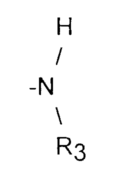 et un éther de formule : 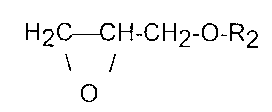 dans laquelle R₂ représente un motif ou un enchaînement de motifs de formule : 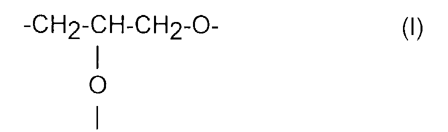 et/ou 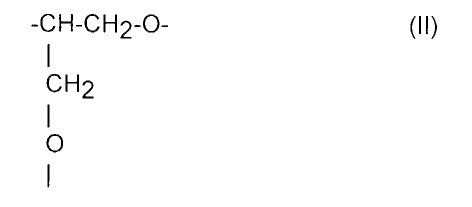 formule dans laquelle la valence disponible de l'atome d'oxygène est liée à un groupement protecteur, et
***b)*** déprotéger;
   pour former les composés de formule : 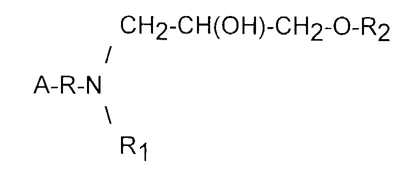 dans laquelle R₁ représente une chaîne hydrocarbonée, linéaire ou ramifiée, saturée ou insaturée, en C₁-C₂₂ ou -CH₂-CH(OH)-CH₂-O-R₂, A représente H ou 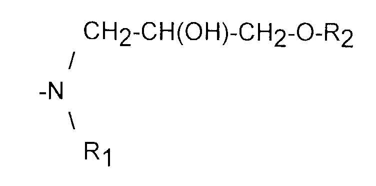 et, R, R₂ ont la signification donnée ci-avant, la valence disponible de l'atome d'oxygène étant liée à un atome d'hydrogène.

Dans la présente invention, l'expression *"valence disponible"* signifie toute valence qui n'est pas engagée dans une liaison avec un motif de formule (I) ou (II).

Lorsque les amines polyglycérylées selon l'invention contiennent plus d'un groupe R₂, ces groupes sont de préférence identiques.

L'amine est généralement choisie parmi les amines saturées telles que la caprylylamine, la caprylamine, la laurylamine, la myristylamine, la cétylamine, la stéarylamine, la béhénylamine, l'éthyl-2-hexylamine, I'isostéarylamine, la N-méthyllaurylamine, la N-méthyl-myristylamine, la N-méthyl-cétylamine ou la N-butyl-octylamine, les amines insaturées telles que l'oléylamine ou l'érucylamine, et les diamines telles que la 1,6-hexanediamine ou la 1,12-dodécanediamine.

De préférence, on utilise les amines dont la chaîne R renferme 8 à 22 atomes de carbone, et mieux encore 10 à 18 atomes de carbone.

De manière avantageuse, on choisit l'amine parmi les composés dans lesquels R₁ est un radical alkyle en C₁-C₄.

On ne sortirait pas du contexte de l'invention en utilisant un mélange des amines précitées, par exemple les amines d'huile de coprah, de palme et de palme kernel, ou de graisse de boeuf hydrogénée ou non hydrogénée.

L'éther est généralement choisi parmi les éthers glycidyles dont R₂ renferme 1 à 7 motifs de formule (I) et/ou (II) et de préférence 1 à 3 motifs, la valence disponible de l'atome d'oxygène desdits motifs étant liée à un groupe protecteur choisi parmi les radicaux alkyles, arylalkyles tels que le benzyle, alkylidènes tels que l'isopropylidène ou acyles tels que l'acétyle.

De préférence, lorsque l'éther glycidyle contient un ou plusieurs motifs de formule (I) ayant deux atomes d'oxygène portant une valence disponible, cet éther présente sous la forme de dérivé acétalique. A titre d'exemple, on peut citer l'éther glycidyle d'isopropylidèneglycérol ou de diisopropylidènetriglycérol.

Les éthers glycidyles selon l'invention peuvent être obtenus de manière conventionnelle par réaction d'épichlorhydrine et d'un alcool de formule HO-R₂ dans laquelle R₂ a la signification donnée précédemment.

Lors de la mise en oeuvre de l'étape a), on utilise généralement un rapport molaire éther/amine compris entre 1 et 4.

Ladite étape est avantageusement effectuée en présence d'un solvant organique permettant d'obtenir un milieu homogène. A titre d'exemples, on peut citer les hydrocarbures linéaires ou ramifiés en C₅ ou C₆, les éthers tels que l'éther de pétrole ou éthylique et les alcools saturés linéaires ou ramifiés en C₁-C₃. De manière avantageuse, on opère au reflux dudit solvant.

L'étape b) de déprotection peut être effectuée de différentes manières selon la nature des groupes protecteurs.

Les amines renfermant des groupes éthers ou esters sont déprotégées selon les méthodes connues de l'homme du métier. A titre d'exemple, on peut citer la déprotection des groupes éthers, notamment benzyliques, par hydrogénation catalytique et celle des groupes esters par saponification.

Les amines renfermant des groupes acétaliques sont hydrolysées par catalyse acide, par exemple en présence d'acide chlorhydrique, et sont généralement traitées par une base afin de les récupérer sous leur forme basique.

A l'issue de l'étape b), on récupère les amines de formule précitée : 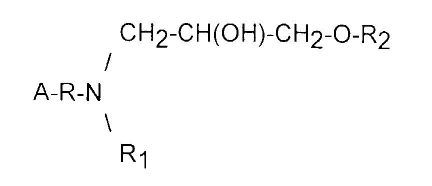 lesquelles amines constituent un objet de la présente invention à l'exception des composés dans lesquels A représente H et R₂ est constitué d'un motif ou d'un enchaînement de motifs de formule -CH₂-CH(OH)-CH₂-O-.

De telles amines peuvent être utilisées dans divers domaines, en particulier ceux pour lesquels des propriétés tensioactives sont requises.

Ces amines peuvent également être transformées en oxyde d'amine, ammonium quaternaire, sel d'ammonium ou bétaïne pouvant être utilisés en tant qu'agent stabilisateur de mousse, détergent notamment dans les shampooings, adoucissant textile ou désinfectant.

Les oxydes d'amine répondent à la formule : 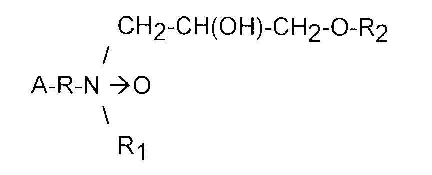 dans laquelle
A représente H ou 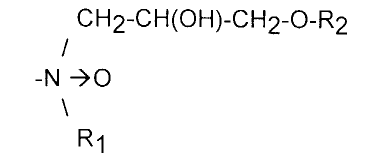
R, R₁ et R₂ ont la signification donnée précédemment,
à l'exception des composés dans lesquels A représente H et R₂ est constitué d'un motif ou d'un enchaînement de motifs de formule -CH₂-CH(OH)-CH₂-O-.

Les oxydes d'amine peuvent être obtenus par réaction des amines selon l'invention et d'un oxydant, par exemple le peroxyde de tertiobutanol en présence d'un catalyseur métallique ou d'eau oxygénée, en milieu aqueux ou hydroalcoolique, l'alcool étant généralement choisi parmi les alcools à haut point éclair. De préférence, on procède en présence d'eau oxygénée et en milieu aqueux.

Les ammoniums quaternaires répondent à la formule : 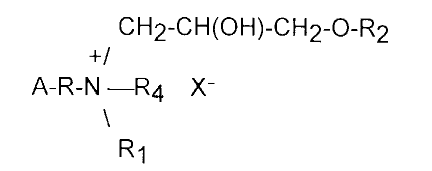 dans laquelle
A représente H ou 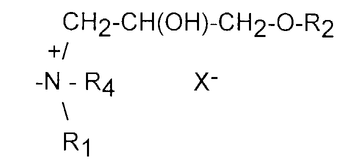 dans laquelle
R, R₁ et R₂ ont la signification donnée précédemment,
R₄ représente un radical alkyle ou arylalkyle,
X représente un atome d'halogène, un groupe alkylphosphate ou un groupe dialkylphosphate,
à l'exception des composés dans lesquels A représente H et R₂ est constitué d'un motif ou d'un enchaînement de motifs de formule -CH₂-CH(OH)-CH₂-O-.

Les ammonium quaternaires préférés sont les composés dans lesquels R₄ et X ont la signification suivante :

| R₄ | X |
|---|---|
| • -CH₃ | Cl |
| • -CH₂-C₆H₅ | Cl |
| • -CH₃ | SO₄(CH₃) |
| • -CH₂-CH₃ | SO₄(CH₂-CH₃) |
| • -CH₃ | PO₄(CH₃)₂ |

Les ammonium quaternaires peuvent être obtenus par réaction des amines selon l'invention et d'un agent de quaternisation conventionnel tel qu'un halogénure d'alkyle, par exemple le chlorure de méthyle, ou d'arylalkyle, par exemple le chlorure de benzyle, les alkylsulfates, par exemple le diméthyl- ou le diéthylsulfate et les alkylphosphates, par exemple le triméthylphosphate. La réaction est généralement mise en oeuvre en milieu aqueux, hydroalcoolique ou alcoolique, par exemple en présence d'un glycol à chaîne courte, d'éthanol ou d'isopropanol.

Les sels d'ammonium répondent à la formule : 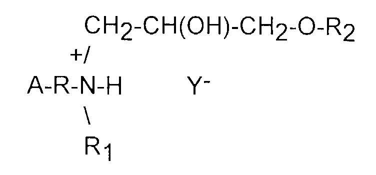 dans laquelle
A représente H ou 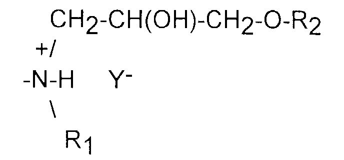
R, R₁ et R₂ ont la signification donnée précédemment,
Y⁻ représente l'anion d'un acide organique ou minéral,
à l'exception des composés dans lesquels A représente H et R₂ est constitué d'un motif ou d'un enchaînement de motifs de formule -CH₂-CH(OH)-CH₂-O-.

Les sels d'ammonium préférés sont les composés dans lesquels Y⁻ représente l'anion formiate, acétate, citrate, chlorure, bromure, phosphate ou sulfate.

Les sels d'ammonium peuvent être obtenus par salification des amines selon l'invention avec un acide organique, par exemple l'acide formique, l'acide acétique et l'acide citrique, ou un acide minéral, par exemple l'acide chlorhydrique, l'acide sulfurique, l'acide phosphorique et l'acide bromhydrique.

Les bétaïnes répondent à la formule : 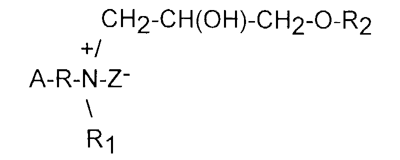 dans laquelle
A représente H ou 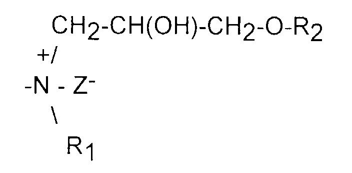
R, R₁ et R₂ ont la signification donnée précédemment,
Z⁻ représente le résidu d'un agent de quaternisation,
à l'exception des composés dans lesquels A représente H et R₂ est constitué d'un motif ou d'un enchaînement de motifs de formule -CH₂-CH(OH)-CH₂-O-.

Les bétaïnes préférées sont les composés dans lesquel Z⁻ représente -CH₂COO⁻, -(CH₂)₂-SO₃⁻, -(CH₂)₃-SO₃⁻, -CH₂-CH(OH)-CH₂-SO₃⁻ ou -(CH₂)₄-SO₃⁻,

Les bétaïnes peuvent être obtenues par réaction des amines selon l'invention et d'un agent de quaternisation tel que l'acide monochloracétique, l'acide chloro-2-éthanesulfonique, l'acide chloro-3-propanesulfonique, l'acide chloro(hydroxy)propane-sulfonique et les sels de ces composés, notamment sodiques ou potassiques, et les sultones, par exemple la 1,3-propane sultone, la 1,4-butane sultone. La réaction est généralement mise en oeuvre en milieu aqueux, hydroalcoolique ou alcoolique, par exemple en présence d'un glycol.

Les exemples qui suivent permettent d'illustrer l'invention. Dans les exemples, on utilise les méthodes d'analyse ci-après.

### Résonancemagnétiquenucléaire (RMN)

Les spectres sont réalisés à 400,13 MHz (¹H) et 100,61 MHz (¹³C).

Les déplacements chimiques sont exprimés en ppm et les constantes de couplage (J) en Hz.

L'aspect des signaux est noté : singulet (s), singulet large (I), triplet (t) et massif (ma).

Le R_{F} est mesuré par chromatographie sur couche mince de silice (60 F₂₅₄ ; MERCK). Les spots de migration sont révélés par pulvérisation d'une solution éthanolique d'acide phosphomolybdique (60 g/l).

### EXEMPLE 1

### a) Préparation de N,N-bis-(3,6'-(2',2'-diméthyl-1',3'-dioxolane-4'-méthyl) (2-hydroxypropyl)éther)-n-octylamine

Dans un ballon bicol de 100 ml muni d'un réfrigérant, on introduit 2,58g (20 mmoles) d'octylamine et 30 ml de méthanol anhydre. On chauffe au reflux du méthanol et on introduit, goutte à goutte 3,76 g (20 mmoles) de 1-O-glycidyl-racisopropylidèneglycérol. Après 2 heures, on ajoute à nouveau 3,76 g de ce dernier composé.

Après 18 heures de réaction, on laisse refroidir le milieu réactionnel et on élimine le méthanol. Le produit de réaction obtenu est séché sous vide à 50°C pendant plusieurs heures.

On récupère 10,08g de N,N-bis-(3,6'-(2',2'-diméthyl-1',3'-dioxalane-4'-méthyl) (2-hydroxypropyl)éther)-n-octylamine dont les caractéristiques sont les suivantes : 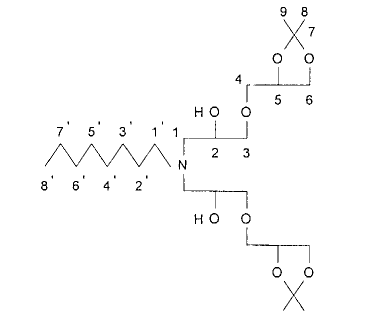
Huile légèrement jaune, translucide.
M = 505,70 g.mol⁻¹ ; C₂₆H₅₁NO₈
Rendement (%) = 100 ; R_{F} (MeOH) = 0,43
¹H RMN (CDCl₃) : 0,88 (t, 3H, H_{8'}) ; 1,21-1,30 (ma, 10H, H₃,→H_{7'}) ; 1,36-1,42 (ma, 2H, H₂,) ; 1,36 (s, 6H, H₈) ; 1,42 (s, 6H, H₉) ; 2,50-2,61 (ma, 6H, H₁ et H_{1'}) ; 3,42-3,60 (ma, 10H, H₂, H₃ et H₄) ; 3,70-3,75 (ma, 2H, H₆ₐ) ; 3,84 (1, 2H, OH) ; 4,04-4,07 (ma, 2H, H_{6b}) ; 4,25-4,30 (ma, 2H, H₅).
¹³C RMN (CDCl₃) : 14,10 (C_{8'}) ; 22,63 (C_{7'}) ; 25,37 (C₈) ; 26,74 (C₉) ; 26,98 ; 27,03 (C_{3'}) ; 27,33 (C₂,) ; 29,29 (C₅,) ; 29,52 (C₄,) ; 31,81 (C₆,) ; 55,64 (C_{1'}) ; 57,32 ; 57,43 ; 57,74 ; 57,85 (C₁) ; 66,57 ; 66,62 (C₆) ; 67,73 ; 67,76 ; 67,81 ; 68,16 ; 68,19 (C₂) ; 72,48 ; 72,52 ; 72,57 ; 74,11 ; 74,14 (C₃ et C₄) ; 74,66 ; 74,73 (C₅) ; 109,41 ; 109,45 (C₇).

### b) Préparation de N,N-bis-(3,1'-(2',3'-dihydroxypropyl) (2-hydroxypropyl)éther)-n-octylamine

Dans un ballon de 100 ml, on introduit 6 g (11,86 mmoles) de N,N-bis-(3,6'-(2',2'-diméthyl-1',3'-dioxolane-4'-méthyl) (2-hydroxypropyl)éther)-n-octylamine et 35,6 ml d'une solution aqueuse d'HCl 1M (3 équivalents). Le mélange est agité à 20°C pendant 3 heures et 30 minutes. Après ajustement du pH à 11 (NaOH 1N), la phase aqueuse est extraite 3 fois par 50 ml d'un mélange acétate d'éthyle/n-butanol (1/1,5 ; v/v). Les phases organiques sont rassemblées et lavées successivement par une solution aqueuse à 5 % en poids de NaHCO₃ (3 fois 30 ml) et à l'eau permutée (30 ml). Les solvants organiques sont éliminés à 40°C sous pression réduite (28 mm Hg), à 50°C sous vide (< 1 mm Hg) et par distillation azéotropique en présence de cyclohexane (traces de n-butanol).

On obtient une huile jaune, trouble et très visqueuse qui est solubilisée dans 150 ml de dichlorométhane et séchée (MgSO₄). Après élimination du solvant, l'huile est solubilisée dans 100 ml de méthanol anhydre et filtrée (papier filtre). Le méthanol est éliminé et, après séchage sous vide à 50°C, on obtient 4,15 g de N,N-bis-(3,1'-(2',3'-dihydroxypropyl) (2-hydroxypropyl)éther)-n-octylamine dont les caractéristiques sont les suivantes : 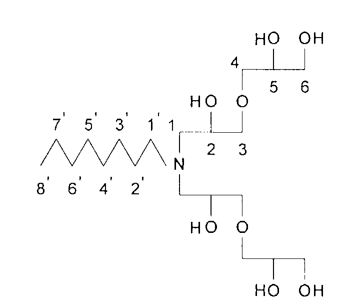
Gel jaune translucide.
M = 425,58 g.mol⁻¹ ; C₂₀H₄₃NO₈
Rendement (%) = 82 ; R_{F} (MeOH/CH₂Cl₂ : 1/1) = 0,61.
¹H RMN (CD₃OD) : 0,81 (t, 3H, H_{8'}) ; 1,16-1,26 (ma, 10H, H_{3'}→H_{7'}) ; 1,34-1,40 (ma, 2H, H_{2'}) ; 2,37-2,54 (ma, 6H, H₁ et H_{1'}) ; 3,31-3,50 (ma, 12H, H₃, H₄ et H₆) ; 3,65-3,75 (ma, 4H, H₂ et H₅).
¹³C RMN (CD₃OD) : 15,31 (C_{8'}) ; 24,56 (C_{7'}) ; 28,91 (C_{3'}) ; 29,40 (C_{2'}) ; 31,31; 31,53 (C_{4'} et C_{5'}) ; 33,87 (C_{6'}) ; 57,87 ; 57,90 (C_{1'}) ; 59,80 ; 60,21 (C₁); 65,28 (C₆) ; 70,43 ; 70,47 ; 70,78 ; 70,82 (C₂) ; 73,13 ; 73,14 (C₅) ; 74,79 ; 74,81 (C₃) ; 75,97 ; 76,00 ; 76,04 (C₄).

### EXEMPLE 2

On procède dans les conditions de l'exemple 1 modifié en ce que l'on utilise la n-décylamine. Les atomes de carbone du radical n-décyle sont numérotés conventionnellement de 1' à 10'.

A l'issue de l'étape a), on obtient la N,N-bis-(3,6'-(2',2'-diméthyl-1',3'-dioxolane-4'-méthyl) (2-hydroxypropyl)éther)-n-décylamine dont les caractéristiques sont les suivantes :
Huile légèrement jaune, translucide.
M = 533,75 g.mol⁻¹ ; C₂₈H₅₅NO₈
Rendement (%) = 100 ; R_{F} (MeOH) = 0,65
¹H RMN (CDCl₃) : 0,88 (t, 3H, H_{10'}) ; 1,20-1,30 (ma, 14H, H_{3'}→H_{9'}) ; 1,35-1,41 (ma, 2H, H_{2'}) ; 1,36 (s, 6H, H₈) ; 1,42 (s, 6H, H₉) ; 2,50-2,60 (ma, 6H, H₁ et H_{1'}) ; 3,42-3,60 (ma, 10H, H₂, H₃ et H₄) ; 3,70-3,76 (ma, 2H, H₆ₐ) ; 3,83 (I, 2H, OH) ; 4,03-4,07 (ma, 2H, H_{6b}) ; 4,26-4,30 (ma, 2H, H₅).
¹³C RMN (CDCl₃) : 14,16 (C_{10'}); 22,71 (C₉,) ; 25,41 (C₈) ; 26,77 (C₉) ; 27,03 ; 27,07 (C_{3'}) ; 27,37 (C_{2'}) ; 29,34 (C_{7'}) ; 29,61 ; 29,67 (C_{4'}→C_{6'}) ; 31,92 (C_{8'}) ; 55,69 (C_{1'}) ; 57,32 ; 57,44 ; 57,74 ; 57,85 (C₁) ; 66,60 ; 66,65 (C₆) ; 67,75 ; 67,80 ; 68,16 (C₂) ; 72,52 ; 72,55 ; 72,61 ; 74,14 (C₃ et C₄) ; 74,70 ; 74,77 (C₅) ; 109,46 ; 109,51 (C₇).

A l'issue de l'étape b), on obtient la N,N-bis-(3,1'-(2',3'-dihydroxypropyl) (2-hydroxypropyl)éther)-n-décylamine dont les caractéristiques sont les suivantes :
Gel jaune translucide.
M = 453,63 g.mol⁻¹ ; C₂₂H₄₇NO₈
Rendement (%) = 80 ; R_{F} (MeOH/CH₂Cl₂ : 1/1) = 0,69
¹H RMN (CD₃OD) : 0,80 (t, 3H, H_{10'}) ; 1,15-1,25 (ma, 14H, H_{3'}→H_{9'}) ; 1,34-1,40 (ma, 2H, H_{2'}) ; 2,37-2,55 (ma, 6H, H₁ et H_{1'}) ; 3,30-3,48 (ma, 12H, H₃, H₄ et H₆) ; 3,65-3,75 (ma, 4H, H₂ et H₅).
¹³C RMN (CD₃OD) : 15,37 (C_{10'}) ; 24,63 (C_{9'}) ; 28,83 ; 28,86 (C_{3'}) ; 29,42 (C_{2'}) ; 31,36 ; 31,63 ; 31,70 (C_{4'}→C_{7'}) ; 33,96 (C_{8'}) ; 57,81; 57,85 (C_{1'}) ; 59,68 ; 60,10 (C₁) ; 65,19 (C₆) ; 70,34 ; 70,39 ; 70,71; 70,75 (C₂) ; 73,11 (C₅) ; 74,71; 74,74 (C₃) ; 75,89 ; 75,93 ; 75,97 (C₄).

### EXEMPLE 3

On procède dans les conditions de l'exemple 1 modifié en ce que l'on utilise la n-dodécylamine. Les atomes de carbone du radical n-dodécyle sont numérotés conventionnellement de 1' à 12'.

A l'issue de l'étape a), on obtient la N,N-bis-(3,6'-(2',2'-diméthyl-1',3'-dioxalane-4'-méthyl) (2-hydroxypropyl)éther)-n-dodécylamine dont les caractéristiques sont les suivantes :
Huile incolore
M = 561,81 g.mol⁻¹ ; C₃₀H₅₉NO₈
Rendement (%) = 100 ; R_{F} (MeOH) = 0,51
¹H RMN (CDCl₃) : 0,88 (t, 3H, H_{12'}) ; 1,20-1,30 (ma, 18H, H_{3'}→H_{11'}) ; 1,36-1,42 (ma, 2H, H_{2'}) ; 1,36 (s, 6H, H₈) ; 1,42 (s, 6H, H₉) ; 2,49-2,59 (ma, 6H, H₁ et H_{1'}) ; 3,43-3,60 (ma, 10H, H₂, H₃ et H₄) ; 3,70-3,76 (ma, 2H, H₆ₐ) ; 3,83 (1, 2H, OH) ; 4,03-4,07 (ma, 2H, H_{6b}) ; 4,26-4,30 (ma, 2H, H₅).
¹³C RMN (CDCl₃) : 14,19 (C_{12'}) ; 22,75 (C_{11'}) ; 25,44 (C₈) ; 26,81 (C₉) ; 27,09; 27,13 (C_{3'}) ; 27,41 (C_{2'}) ; 29,41 (C_{9'}) ; 29,65; 29,70 (C_{4'}→C_{8'}) ; 31,97 (C_{10'}) ; 55,72 (C_{1'}) ; 57,34 ; 57,46 ; 57,76 ; 57,87 (C₁) ; 66,64 ; 66,69 (C₆) ; 67,78 ; 67,83 ; 68,16 (C₂) ; 72,56 ; 72,60 ; 72,66 ; 74,15 ; 74,19 (C₃ et C₄) ; 74,73 ; 74,80 (C₅) ; 109,49 ; 109,54 (C₇).

A l'issue de l'étape b), on obtient la N,N-bis-(3,1'-(2',3'-dihydroxypropyl) (2-hydroxypropyl)éther)-n-dodécylamine dont les caractéristiques sont les suivantes :
Gel jaune translucide.
M = 481,69 g.mol⁻¹ ; C₂₄H₅₁NO₈
Rendement (%) = 84 ; R_{F} (MeOH/CH₂Cl₂ : 1/1) = 0,57
¹H RMN (CD₃OD) : 0,80 (t, 3H, H_{12'}) ; 1,14-1,24 (ma, 18H, H_{3'}→H_{11'}) ; 1,34-1,40 (ma, 2H, H_{2'}) ; 2,37-2,54 (ma, 6H, H₁ et H_{1'}) ; 3,32-3,50 (ma, 12H, H₃, H₄ et H₆) ; 3,65-3,75 (ma, 4H, H₂ et H₅).
¹³C RMN (CD₃OD) : 15,39 (C_{12'}) ; 24,63 (C_{11'}) ; 28,85 ; 28,88 (C_{3'}) ; 29,43 (C_{2'}) ; 31,37 ; 31,66 ; 31,69 (C_{4'}→C_{9'}) ; 33,96 (C_{10'}); 57,81 ; 57,84 (C_{1'}) ; 59,67 ; 60,09 (C₁) ; 65,18 (C₆) ; 70,34 ; 70,39 ; 70,72 ; 70,76 (C₂) ; 73,09 ; 73,10 (C₅) ; 74,70 ; 74,73 (C₃) ; 75,88 ; 75,92 ; 75,96 (C₄).

### EXEMPLE 4

On procède dans les conditions de l'exemple 1 modifié en ce que l'on utilise la n-tétradécylamine. Les atomes de carbone du radical n-tétradécyle sont numérotés conventionnellement de 1' à 14'.

A l'issue de l'étape a), on obtient la N,N-bis-(3,6'-(2',2'-diméthyl-1',3'-dioxolane-4'-méthyl) (2-hydroxypropyl)éther)-n-tétradécylamine dont les caractéristiques sont les suivantes :
Huile incolore
M = 589,86 g.mol⁻¹ ; C₃₂H₆₃NO₈
Rendement (%) = 100 ; R_{F} (MeOH) = 0,47
¹H RMN (CDCl₃) : 0,88 (t, 3H, H_{14'}) ; 1,20-1,30 (ma, 22H, H_{3'}→H_{13'}) ; 1,35-1,41 (ma, 2H, H_{2'}) ; 1,36 (s, 6H, H₈) ; 1,42 (s, 6H, H₉) ; 2,50-2,59 (ma, 6H, H₁ et H_{1'}) ; 3,41-3,60 (ma, 10H, H_{2'} H₃ et H₄) ; 3,70-3,75 (ma, 2H, H₆ₐ) ; 3,84 (1, 2H, OH) ; 4,03-4,07 (ma, 2H, H_{6b}) ; 4,26-4,30 (ma, 2H, H₅).
¹³C RMN (CDCl₃) : 14,17 (C_{14'}) ; 22,72 (C_{13'}) ; 25,41 (C₈) ; 26,78 (C₉) ; 27,04 ; 27,09 (C_{3'}) ; 27,39 (C_{2'}) ; 29,39 (C_{11'}) ; 29,63 ; 29,69 ; 29,72 (C_{4'}→C_{10'}) ; 31,95 (C₁₂,) ; 55,69 (C_{1'}) ; 57,33 ; 57,45 ; 57,75 ; 57,87 (C₁) ; 66,61; 66,65 (C₆) ; 67,79; 68,19 (C₂) ; 72,52; 72,56 ; 72,62; 74,14; 74,17 (C₃ et C₄) ; 74,70; 74,77 (C₅) ; 109,46 ; 109,51 (C₇).

A l'issue de l'étape b), on obtient la N,N-bis-(3,1'-(2',3'-dihydroxypropyl) (2-hydroxypropyl)éther)-n-tétradecylamine dont les caractéristiques sont les suivantes :
Gel opaque.
M = 509,74 g.mol⁻¹ ; C₂₆H₅₅NO₈
Rendement (%) = 80 ; R_{F} (MeOH/CH₂Cl₂ : 1/1) = 0,51
¹H RMN (CD₃OD) : 0,81 (t, 3H, H_{14'}) ; 1,14-1,24 (ma, 22H, H_{3'}→H_{13'}) ; 1,34-1,40 (ma, 2H, H_{2'}) ; 2,37-2,54 (ma, 6H, H₁ et H_{1'}) ; 3,31-3,50 (ma, 12H, H₃, H₄ et H₆) ; 3,65-3,75 (ma, 4H, H₂ et H₅).
¹³C RMN (CD₃OD) : 15,39 (C_{14'}) ; 24,64 (C_{13'}) ; 28,87 ; 28,90 (C_{3'}) ; 29,44 (C_{2'}) ; 31,39 ; 31,67 ; 31,69 ; 31,70 (C_{4'}→C_{11'}) ; 33,97 (C_{12'}) ; 57,83 ; 57,87 (C_{1'}) ; 59,69 ; 60,11 (C₁) ; 65,19 (C₆) ; 70,35 ; 70,40 ; 70,73 ; 70,77 (C₂) ; 73,10 (C₅) ; 74,72 ; 74,75 (C₃) ; 75,89 ; 75,93 ; 75,97 (C₄).

### EXEMPLE 5

On procède dans les conditions de l'exemple 1 modifié en ce que l'on utilise la n-octadécylamine. Les atomes de carbone du radical n-octadécyle sont numérotés conventionnellement de 1' à 18'.

A l'issue de l'étape a), on obtient la N,N-bis-(3,6'-(2',2'-diméthyl-1',3'-dioxalane-4'-méthyl) (2-hydroxypropyl)éther)-n-octadécylamine dont les caractéristiques sont les suivantes :
Huile jaune translucide.
M = 645,97 g.mol⁻¹ ; C₃₆H₇₁NO₈
Rendement (%) = 100 ; R_{F} (MeOH) = 0,54
¹H RMN (CDCl₃) : 0,88 (t, 3H, H_{18'}) ; 1,19-1,31 (ma, 30H, H_{3'}→H_{17'}) ; 1,35-1,41 (ma, 2H, H_{2'}) ; 1,36 (s, 6H, H₈) ; 1,42 (s, 6H, H₉) ; 2,49-2,60 (ma, 6H, H₁ et H_{1'}) ; 3,42-3,60 (ma, 10H, H_{2'} H₃ et H₄) ; 3,70-3,75 (ma, 2H, H₆ₐ) ; 3,84 (1, 2H, OH) ; 4,03-4,07 (ma, 2H, H_{6b}) ; 4,25-4,30 (ma, 2H, H₅).
¹³C RMN (CDCl₃) : 14,15 (C_{18'}) ; 22,71 (C_{17'}) ; 25,40 (C₈) ; 26,76 (C₉) ; 27,03 ; 27,07 (C_{3'}) ; 27,38 (C_{2'}) ; 29,38 (C_{15'}) ; 29,62 ; 29,68 ; 29,72 (C_{4'}→C_{14'}) ; 31,94 (C_{16'}) ; 55,67 (C_{1'}) ; 57,34 ; 57,46 ; 57,77 ; 57,88 (C₁) ; 66,60 ; 66,64 (C₆) ; 67,79 ; 67,81 ; 67,87 ; 68,22 (C₂) ; 72,51 ; 72,55 ; 72,60 ; 74,14 ; 74,17 (C₃ et C₄) ; 74,69 ; 74,76 (C₅) ; 109,44 ; 109,48 (C₇).

A l'issue de l'étape b), on obtient la N,N-bis-(3,1'-(2',3'-dihydroxypropyl) (2-hydroxypropyl)éther)-n-octadécylamine dont les caractéristiques sont les suivantes :
Gel jaune opaque.
M = 565,85 g.mol⁻¹ ; C₃₀H₆₃NO₈
Rendement (%) = 95 ; R_{F} (MeOH/CH₂Cl₂ : 1/1) = 0,57
¹H RMN (CD₃OD) : 0,80 (t, 3H, H_{18'}) ; 1,13-1,25 (ma, 30H, H_{3'}→H_{17'}) ; 1,35-1,41 (ma, 2H, H_{2'}) ; 2,40-2,57 (ma, 6H, H₁ et H_{1'}) ; 3,31-3,51 (ma, 12H, H₃, H₄ et H₆) ; 3,65-3,76 (ma, 4H, H₂ et H₅).
¹³C RMN (CD₃OD) : 15,32 (C_{18'}) ; 24,59 (C_{17'}) ; 28,82 (C_{3'}) ; 29,40 (C_{2'}) ; 31,32 ; 31,60 ; 31,62 ; 31,65 (C_{4'}→C_{15'}) ; 33,93 (C_{16'}) ; 57,87 ; 57,91 (C_{1'}) ; 59,77 ; 60,18 (C₁) ; 65,28 (C₆) ; 70,30 ; 70,34 ; 70,66 ; 70,70 (C₂) ; 73,13 ; 73,15 (C₅) ; 74,81 ; 74,83 (C₃) ; 75,96 ; 75,99 ; 76,03 (C₄).

### EXEMPLE 6

Dans un ballon bicol de 50 ml muni d'un réfrigérant, on introduit 0,61 g (3,22 mmoles) de n-dodécylamine et 10 ml de méthanol anhydre. On chauffe au reflux du méthanol et on introduit, goutte à goutte, 1,21g (3,22 mmoles) de 1-O-glycidyl-rac-diisopropylidènetriglycérol. Après deux heures, on ajoute à nouveau 1,21 g de ce dernier composé.

Après 20 heures de réaction, on laisse refroidir le milieu réactionnel et on élimine le méthanol. Le produit de réaction obtenu est séché sous vide à 50°C pendant plusieurs heures.

On récupère 2,92 g de N,N-bis-(3,5'-(2-hydroxypropyl)(diisopropylidène-triglycéryl)éther)-n-dodécylamine sous la forme d'une huile jaunâtre. A 2,85 g (3,04 mmoles) de ce dernier composé, on ajoute 18,2 ml d'une solution aqueuse d'HCl 1N (6 équivalents).

Le mélange est agité à 20°C pendant 3 heures. Après ajustement du pH du milieu réactionnel à 11 (NaOH 5N), on ajoute 100 ml de toluène et on élimine l'eau par distillation azéotropique. Le résidu obtenu est repris par 20 ml de méthanol anhydre et filtré sur du sable de Fontainebleau. La filtration est répétée jusqu'à ce que la solution méthanolique devienne limpide à 0°C (faible présence de sels). Après séchage sous vide à 50°C pendant plusieurs heures, on récupère un gel incolore de N,N-bis-(3,5'-(2-hydroxypropyl) (triglycéryl)éther)-n-dodécylamine ayant les caractéristiques suivantes : 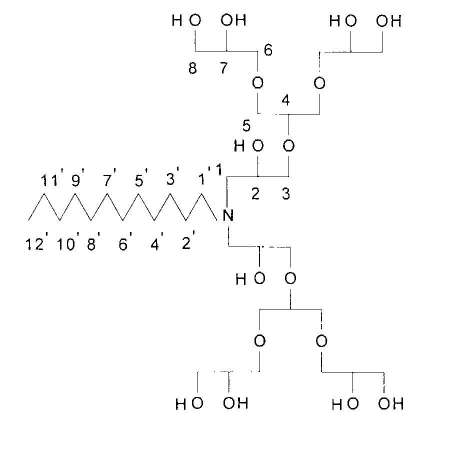
Gel incolore.
M = 777,99 g.mol⁻¹ ; C₃₆H₇₅NO₁₆
Rendement (%) = 73 ; R_{F} (MeOH) = 0,39
¹H RMN (CD₃OD) : 0,80 (t, 3H, H_{12'}) ; 1,14-1,24 (ma, 18H, H_{3'}→H_{11'}) ; 1,35-1,41 (ma, 2H, H_{2'}) ; 2,37-2,56 (ma, 6H, H₁ et H_{1'}) ; 3,34-3,74 (ma, 36H, H₂→ H₈).
¹³C RMN (CD₃OD) : 15,36 (C_{12'}) ; 24,61 (C_{11'}) ; 28,47 (C_{3'}) ; 29,35 ; 29,37 (C₂,) ; 31,36 ; 31,61 ; 31,65 ; 31,66 ; 31,69 (C_{4'}→C_{9'}) ; 33,95 (C_{10'}); 57,74 (C_{1'}) ; 59,59 ; 60,01 (C₁) ; 65,28 (C₈) ; 70,14 ; 70,51 (C₂) ; 73,01 (C₇) ; 73,11 ; 73,15 (C₅) ; 74,77 ; 74,80 (C₆) ; 74,87 ; 74,96 (C₃) ; 80,58 (C₄).

### EXEMPLE 7

On procède dans les conditions de l'exemple 6 modifié en ce que l'on utilise la n-tétradécylamine. Les atomes de carbone du radical n-tétradécyle sont numérotés conventionnellement de 1' à 14'.

A l'issue de l'étape a), on obtient la N,N-bis-(3,5'-(2-hydroxypropyl) (triglycéryl)éther)-n-tétradécylamine dont les caractéristiques sont les suivantes :
Gel légèrement jaune.
M = 806,05 g.mol⁻¹ ; C₃₈H₇₉NO₁₆
Rendement (%) = 85 ; R_{F} (MeOH) = 0,38
¹H RMN (CD₃OD) : 0,81 (t, 3H, H_{14'}) ; 1,15-1,25 (ma, 22H, H_{3'}→H_{13'}) ; 1,34-1,40 (ma, 2H, H_{2'}) ; 2,36-2,56 (ma, 6H, H₁ et H_{1'}) ; 3,33-3,75 (ma, 36H, H₂→ H₈).
¹³C RMN (CD₃OD) : 15,36 (C₁₄,) ; 24,65 (C_{13'}) ; 28,88 (C_{3'}) ; 29,49 (C_{2'}) ; 31,39 ; 31,68 ; 31,72 ; 31,73 ; 31,76 ; 31,98 (C_{4'}→C_{11'}) ; 33,98 (C_{12'}) ; 57,89 (C_{1'}) ; 59,79 ; 60,16 (C₁) ; 65,32 (C₈) ; 70,64 ; 70,96 (C₂) ; 73,07 (C₇) ; 73,15 ; 73,20 (C₅) ; 74,81 ; 74,85 (C₆) ; 75,05 ; 75,12 (C₃) ; 80,65 ; 80,67 (C₄).

### EXEMPLE 8

On procède dans les conditions de l'exemple 6 modifié en ce que l'on utilise la n-octadécylamine. Les atomes de carbone du radical n-octadécyle sont numérotés conventionnellement de 1' à 18'.

A l'issue de l'étape a), on obtient la N,N-bis-(3,5'-(2-hydroxypropyl) (triglycéryl)éther)-n-octadécylamine dont les caractéristiques sont les suivantes :
Mousse hygroscopique légèrement jaune.
M = 862,15 g.mol⁻¹ ; C₄₂H₈₇NO₁₆
Rendement (%) = 81 ; R_{F} (MeOH) = 0,39
¹H RMN (CD₃OD) : 0,81 (t, 3H, H_{18'}) ; 1,13-1,25 (ma, 30H, H_{3'}→H_{17'}) ; 1,34-1,41 (ma, 2H, H_{2'}) ; 2,38-2,56 (ma, 6H, H₁ et H_{1'}) ; 3,34-3,76 (ma, 36H, H₂→ H₈).
¹³C RMN (CD₃OD) : 15,37 (C_{18'}) ; 24,63 (C_{17'}) ; 28,79 ; 28,83 (C_{3'}) ; 29,48 (C_{2'}) ; 31,37 ; 31,65 ; 31,69 ; 31,75 (C_{4'}→C_{15'}) ; 33,96 (C_{16'}) ; 57,84 (C_{1'}) ; 59,61 ; 60,09 (C₁) ; 65,23 (C₈) ; 70,47 ; 70,84 (C₂) ; 72,98 (C₇) ; 73,05 ; 73,10 (C₅) ; 74,75 ; 74,78 (C₆) ; 74,87 ; 74,94 (C₃) ; 80,49 ; 80,56 (C₄).

## Revendications

1. Procédé de préparation d'amines de formule : 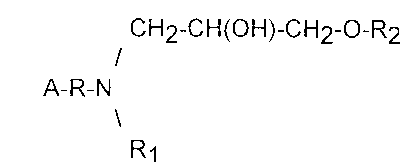 dans laquelle R représente une chaîne hydrocarbonée, linéaire ou ramifiée, saturée ou insaturée, en C₆-C₃₂, R₁ représente une chaîne hydrocarbonée, linéaire ou ramifiée, saturée ou insaturée, en C₁-C₂₂ ou -CH₂-CH(OH)-CH₂-O-R₂, A représente H ou 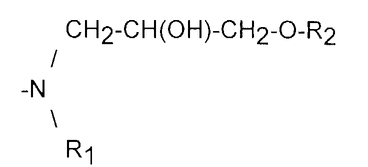 R₂ représente un motif ou un enchaînement de motifs de formule : 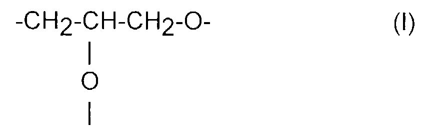 et/ou 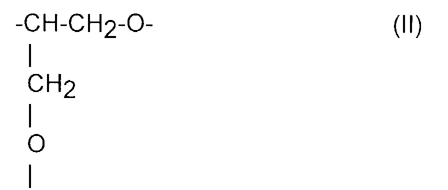 formule dans laquelle la valence disponible de l'atome d'oxygène est liée à un atome d'hydrogène,
ledit procédé étant caractérisé en ce qu'il consiste à :
***a***) faire réagir une amine de formule 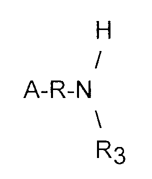 et un éther de formule : 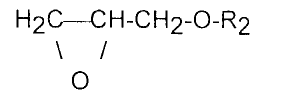 dans laquelle R₃ représente H ou une chaîne hydrocarbonée, linéaire ou ramifiée, saturée ou insaturée, en C₁-C₂₂, et A, R, R₂ ont la signification donnée ci-avant, la valence disponible de l'atome d'oxygène étant liée à un groupement protecteur, et
***b)*** déprotéger.

2. Procédé selon la revendication 1, caractérisé en ce que R renferme 8 à 22 atomes de carbone.

3. Procédé selon la revendication 1, caractérisé en ce que R₁ renferme 1 à 4 atomes de carbone.

4. Procédé selon l'une des revendications 1 à 3, caractérisé en ce que l'éther est choisi parmi les éthers glycidyles dont R₂ renferme 1 à 7 motifs de formule (I) et/ou (II).

5. Procédé selon l'une des revendications 1 à 4, caractérisé en ce que le groupe protecteur est choisi parmi les radicaux alkyles, arylalkyles, alkylidènes ou acyles.

6. Amine polyglycérylée de formule : 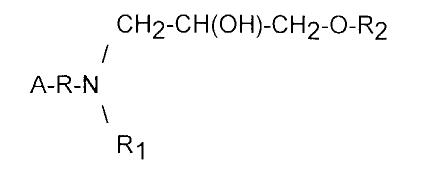 dans laquelle R représente une chaîne hydrocarbonée, linéaire ou ramifiée, saturée ou insaturée, en C₆-C₃₂, R₁ représente une chaîne hydrocarbonée, linéaire ou ramifiée, saturée ou insaturée, en C₁-C₂₂ ou -CH₂-CH(OH)-CH₂-O-R₂, A représente H ou 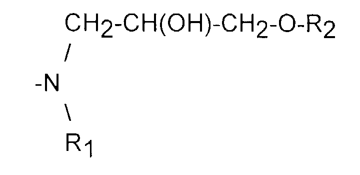 R₂ représente un motif ou un enchaînement de motifs de formule : 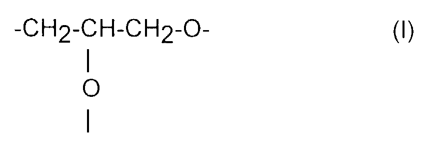 et/ou 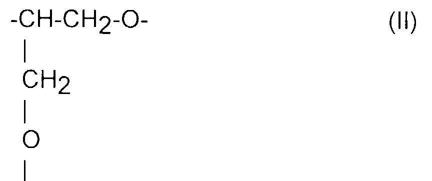 formule dans laquelle la valence disponible de l'atome d'oxygène est liée à un atome d'hydrogène,
à l'exception des composés dans lesquels A représente H et R₂ est constitué d'un motif ou d'un enchaînement de motifs de formule -CH₂-CH(OH)-CH₂-O-.

7. Amines selon la revendication 6, caractérisées en ce que R renferme 8 à 22 atomes de carbone.

8. Amines selon la revendication 6 ou 7, caractérisées en ce que R₁ renferme 1 à 4 atomes de carbone.

9. Amines selon l'une des revendications 6 à 8, caractérisées en ce que R₂ renferme 1 à 7 motifs de formule (I) et/ou (II).

10. Oxyde d'amine de formule : 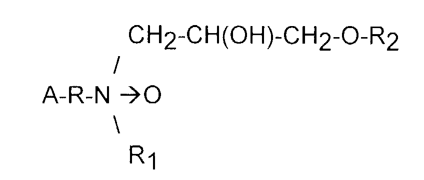 dans laquelle
A représente H ou 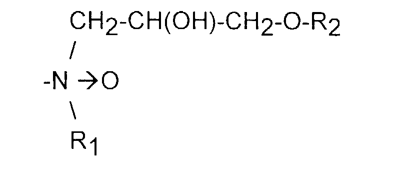
R, R₁ et R₂ ont la signification donnée précédemment,
à l'exception des composés dans lesquels A représente H et R₂ est constitué d'un motif ou d'un enchaînement de motifs de formule -CH₂-CH(OH)-CH₂-O-.

11. Ammonium quaternaire de formule : 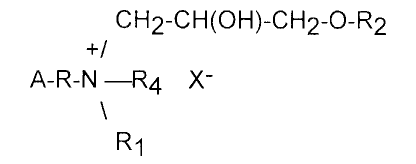 dans laquelle
A représente H ou 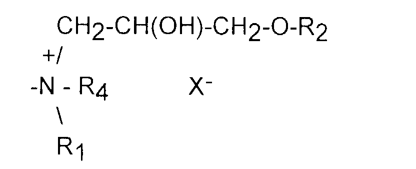 dans laquelle
R, R₁ et R₂ ont la signification donnée précédemment,
R₄ représente un radical alkyle ou arylalkyle,
X représente un atome d'halogène, un groupe alkylphosphate ou un groupe dialkylphosphate,
à l'exception des composés dans lesquels A représente H et R₂ est constitué d'un motif ou d'un enchaînement de motifs de formule -CH₂-CH(OH)-CH₂-O-.

12. Ammonium quaternaire selon la revendication 11, dans lequel R₄ et X représentent respectivement :
| | |
|---|---|
| • -CH₃ | Cl |
| • -CH₂-C₆H₅ | Cl |
| • -CH₃ | SO₄(CH₃) |
| • -CH₂-CH₃ | SO₄(CH₂-CH₃) |
| • -CH₃ | PO₄(CH₃)₂ |

13. Sel d'ammonium de formule : 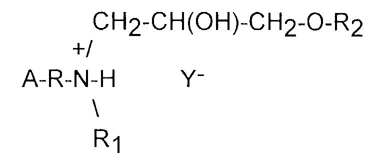 dans laquelle
A représente H ou 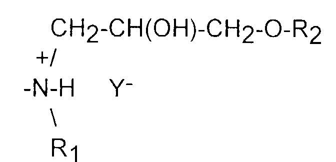
R, R₁ et R₂ ont la signification donnée précédemment,
Y représente l'anion d'un acide organique ou minéral,
à l'exception des composés dans lesquels A représente H et R₂ est constitué d'un motif ou d'un enchaînement de motifs de formule -CH₂-CH(OH)-CH₂-O-.

14. Sel selon la revendication 13, dans lequel Y⁻ représente l'anion formiate, acétate, citrate, chlorure, bromure, phosphate ou sulfate.

15. Bétaïne de formule : 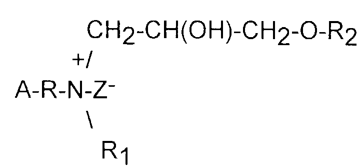 dans laquelle
A représente H ou 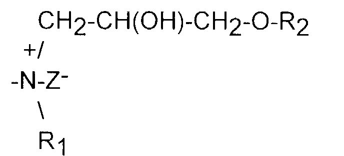
R, R₁ et R₂ ont la signification donnée précédemment,
Z⁻ représente le résidu d'un agent de quaternisation,
à l'exception des composés dans lesquels A représente H et R₂ est constitué d'un motif ou d'un enchaînement de motifs de formule -CH₂-CH(OH)-CH₂-O-.

16. Bétaïne selon la revendication 15, dans laquelle Z- représente -CH₂COO⁻, -(CH₂)₂-SO₃⁻, -(CH₂)₃-SO₃⁻, -CH₂-CH(OH)-CH₂-SO₃⁻ ou -(CH₂)₄-SO₃⁻.
